# EUROPEAN PATENT SPECIFICATION

(11) **EP 2 686 038 B1**
(45) Date of publication and mention of the grant of the patent: **16.11.2022**
(21) Application number: 12719107.0
(22) Date of filing: 14.03.2012
(51) Int. Cl.: A61M 5/142

(54) **INSTRUMENT FOR FILLING AN IMPLANTED DRUG PUMP**
INSTRUMENT ZUM FÜLLEN EINER IMPLANTIERTEN WIRKSTOFFPUMPE
INSTRUMENT PERMETTANT DE REMPLIR UNE POMPE À MÉDICAMENTS IMPLANTÉE

(30) Priority: 14.03.2011 US 201161452399 P
(43) Date of publication of application: 22.01.2014
(73) Proprietor: MiniPumps, LLC, Pasadena, CA 91107 (US)
(72) Inventor: SHIH, Jason, Yorba Linda CA 92886 (US); CAFFEY, Sean, Manhattan Beach CA 90266 (US); HUMAYUN, Mark, Glendale CA 91206 (US); JIANG, Fukang, Pasadena CA 91125 (US); PANG, Changlin, Pasadena CA 91106 (US); PECK, Raymond, Los Angeles CA 90032 (US); TAI, Yu-Chong, Pasadena CA 91106 (US)
(74) Representative: Marks & Clerk LLP
(86) International application number: PCT/US2012/029029
(87) International publication number: WO 2012/125695

(56) References cited:
- WO-A1-00/74751
- WO-A1-2011/022484
- WO-A2-2009/137777

## Description

### FIELD OF THE INVENTION

In various embodiments, the present invention relates, generally, to implantable drug pump devices, and in particular to systems for refilling such devices.

### BACKGROUND

Medical treatment often requires the administration of a therapeutic agent (e.g., medicament, drugs, etc.) to a particular part of a patient's body. As patients live longer and are more frequently diagnosed with chronic and often debilitating ailments, the result will be an increase in the need to place protein therapeutics, small-molecule drugs and other medications into targeted areas throughout the body. Some maladies, however, are difficult to treat with currently available therapies and/or require administration of drugs to anatomical regions to which access is difficult to achieve.

A patient's eye is a prime example of a difficult-to-reach anatomical region, and many vision-threatening diseases, including retinitis pigmentosa, age-related macular degeneration (AMD), diabetic retinopathy, and glaucoma, are incurable and yet difficult to treat with currently available therapies. For example, oral medications have systemic side effects; topical applications may sting and engender poor compliance; injections require a medical visit, can be painful and risk infection; and sustained-release implants must typically be removed after their supply is exhausted (and generally offer limited ability to change the dose in response to the clinical situation). Another example is cancer, such as breast cancer or meningiomas, where large doses of highly toxic chemotherapies such as rapamycin or irinotecan (CPT-11) are typically administered to the patient intravenously, resulting in numerous undesired side effects outside the targeted area. Yet another example is drug delivery to the knee, where drugs often have difficulty penetrating the avascular cartilage tissue for diseases such as osteoarthritis.

Implantable drug delivery systems, which may have a refillable drug reservoir, cannula and check valve, etc., generally allow for controlled delivery of pharmaceutical solutions to a specified target. As drug within the drug reservoir depletes, the physician can refill the reservoir with, for example, a syringe, via a refill port while leaving the device implanted within the patient's body. This approach can minimize the surgical incision needed for implantation and avoids future or repeated invasive surgery or procedures. WO2009/137777 discloses a refilling tool as defined in the preamble of claim 1.

Conventionally, the drug pump is refilled manually using, for example, a handheld syringe. This approach can be both inconvenient and dangerous. It is typically difficult to monitor the pressure in the syringe, and a large pressure may be generated inadvertently
particularly when small volumes are involved and the syringe plunger is of small diameter. Excessive pressures can damage the pump and/or cause improper drug expulsion. Additionally, refilling the implantable pump may represent a difficult manual task. For example, the pump's refill port, which is usually located on the pump surface to facilitate post-implantation access, may be inaccessible or inconvenient to access due to the recipient's
internal anatomy. This makes the refill procedure uncomfortable for the recipient and, once again, risks damage to the pump. Refilling difficulties are especially acute if the therapeutic procedure involves a multiple-drug administration which requires several cycles of needle insertion and withdrawal as different fluids are removed and injected into the pump, causing stress for both the patient and doctor and creating wear on the refill port.

Consequently, there is a need for a refilling system that can monitor the pressure during drug administration and reduce the insertion frequency into the implanted pump.

### SUMMARY

According to the present invention there is provided a tool for refilling an implantable pump as set out in claim 1.

In accordance with various embodiments of the invention described herein, a dedicated instrument is used to automatically facilitate a filling or refilling therapeutic
procedure via, for example, a self-sealing needle-accessible refill port in an implanted drug pump. The procedure may include, for example, emptying, rinsing, and filling the drug reservoir. An instrument in accordance with the current invention may be configured such that only a single needle insertion in the fluid access refill port is required to direct multiple fluids to the implanted pump. Additionally, the instrument may include a valve, a pressure sensor
and/or a flow sensor to detect and control the pressure therein. The automation and/or pressure-control of the refilling process protects pump components (e.g., the refill port or the reservoir) from potential damage and ensures reliable and repeatable drug filling.

Accordingly, in one aspect, the invention pertains to a tool for refilling an implantable pump having at least one reservoir. In various embodiments, the tool includes: (i) multiple independent fluid channels, (ii) a fluid reservoir in fluid communication with a first fluid channel, and (iii) a connector for removably connecting the fluid channels to the at least one reservoir. The tool may include one or more pumps that are fluidly coupled to the fluid channels. The pump(s) may be configured to apply positive pressure to the first fluid channel so as to drive fluid from the fluid reservoir therethrough, and to apply negative pressure to a second fluid channel.

The pump(s) and the independent fluid channels may differ from each other in number. For example, when there are one pump and at least two independent fluid channels, the pump may be configured to generate suction through a first one of the channels and to drive a fluid through a second one of the channels. If there is a third independent fluid channel connected to the pump, the pump may be configured to drive a second fluid through the third fluid channel.

The tool may include a sensor associated with each of the channels for monitoring at least one parameter relating to liquid flowing therethrough. The sensor may be a flow sensor and/or a pressure sensor and the parameter may be a flow rate of the liquid and/or pressure in the channels, respectively.

Additionally, the tool may include governing circuitry for preventing the monitored parameter from exceeding or falling below a predefined level. Two valves that are responsive to the governing circuitry may be used for controlling fluid flow through the first and second fluid channels.

In a second aspect, the invention relates to a tool for refilling an implantable pump having a reservoir. The tool may include a refilling kit and a base unit. The refilling kit may have two independent fluid channels connectable at one end thereof to the reservoir. The base, which is removably connectable to the other ends of the fluid channels, may include at least one pump, a sensor for monitoring at least one parameter relating to flow through the fluid channels, and feedback circuitry for controlling flow through the fluid channels based on the monitored parameter(s).

The refilling kit may be connectable to the reservoir by means, for example, of a needle configured for entering the reservoir. The needle may have separate lumens each fluidly coupled to one of the fluid channels. The tool may further include a locking system that is associated with the refilling kit for preventing injection of an unapproved fluid into the reservoir.

Reference throughout this specification to "one example," "an example," "one embodiment," or "an embodiment" means that a particular feature, structure, or characteristic described in connection with the example is included in at least one example of the present technology. Thus, the occurrences of the phrases "in one example," "in an example," "one embodiment," or "an embodiment" in various places throughout this specification are not necessarily all referring to the same example. Furthermore, the particular features, structures, routines, steps, or characteristics may be combined in any suitable manner in one or more examples of the technology. The headings provided herein are for convenience only and are not intended to limit or interpret the scope or meaning of the claimed technology.

### BRIEF DESCRIPTION OF THE DRAWINGS

In the drawings, like reference characters generally refer to the same parts throughout the different views. Also, the drawings are not necessarily to scale, with an emphasis instead generally being placed upon illustrating the principles of the invention. In the following description, various embodiments of the present invention are described with reference to the following drawings, in which:
FIG. 1 schematically illustrates an implantable drug-delivery pump;
FIG. 2A schematically illustrates a filling/refilling instrument in accordance with an embodiment of the current invention;
FIG. 2B schematically depicts the filling/refilling instrument incorporating valves, sensors and a feedback system;
FIG. 2C schematically illustrates a single pump associated with two fluid channels;
FIG. 2D schematically depicts a base unit having pumps, sensors, valves and a feedback system and a filling/refilling kit having fluid channels;
FIG. 3 schematically depicts the wash channel and the filling channel merged into a single channel; and
FIG. 4 schematically depicts two lumens in the needle that is used to puncture the drug reservoir.

### DETAILED DESCRIPTION

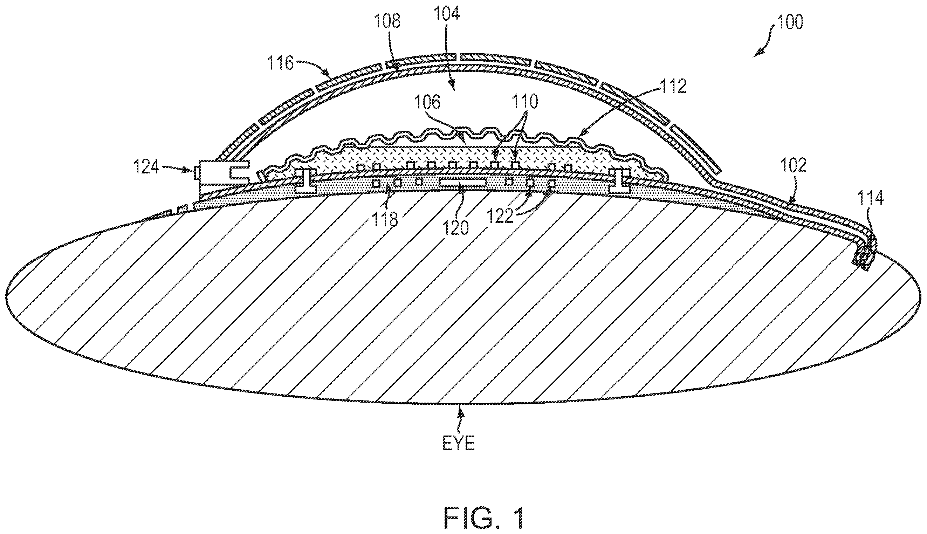

Refer first to FIG. 1, which illustrates an electrolysis-actuated, implantable drug-delivery pump 100, as described, for example, in U.S. Serial No. 12/463,251. As illustrated, the implantable drug pump 100 has a cannula 102 and a pair of chambers 104, 106 bounded by an envelope 108. The top chamber 104 defines a drug reservoir that contains the drug to be administered in liquid form, and the bottom chamber 106 contains a liquid which, when subjected to electrolysis using electrolysis electrodes 110, evolves a gaseous product. The two chambers are separated by a corrugated diaphragm 112. The cannula 102 connects the top drug chamber 104 with a check valve 114 inserted at the site of administration. The envelope 108 resides within a shaped protective shell 116 made of a flexible material (e.g., a bladder or collapsible chamber) or a relatively rigid biocompatible material (e.g medical-grade polypropylene). Control circuitry 118, a battery 120 and an induction coil 122 for power and data transmission are embedded under the parylene chambers (i.e., between the bottom wall of the electrolyte chamber 106 and the floor of the shell 116). One or more refill ports 124 are in fluid communication with the drug reservoir 104 and permit the drug reservoir 104 to be refillable by inserting, for example, a refill needle (not shown) therethrough. The refill port 124 may include a self-sealing material such that the needle can puncture the top surface thereof and the surface reseals itself upon removal of the needle. The self-sealing material may be able to withstand multiple punctures by the needle, and is biocompatible. Through the refill port 124, the existing fluid in the reservoir can be removed, the reservoir washed, and a filling/refilling solution injected. Certain embodiments of the invention involve an external device that can be interfaced to the liquid containing reservoir for the automatic filling/refilling of the reservoir.

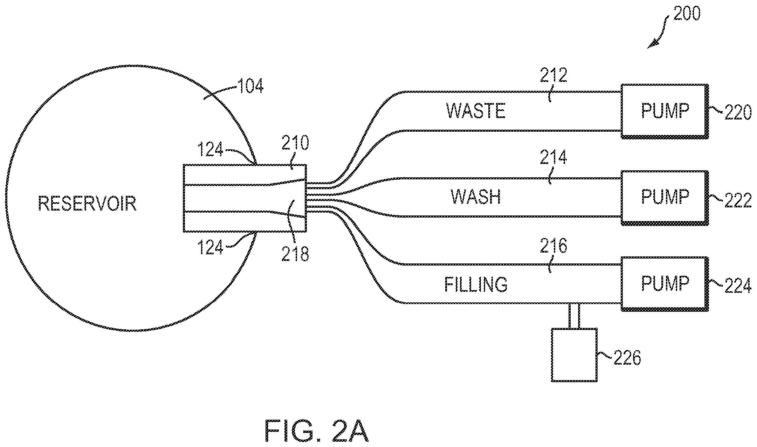

FIG. 2A depicts an instrument 200 that interfaces with and refills, for example, the implantable drug-delivery pump 100 as show in FIG. 1 in accordance with an embodiment of the current invention. The instrument 200 may include a needle 210 piercing through the surface of the refill port 124 to facilitate the fluid communication between the drug reservoir 104 in the implantable pump 100 and the instrument 200 that has one or more independent fluid channels 212, 214, 216. In various embodiments, the refilling process begins with removing or aspirating an expired and/or remnant fluid from the drug reservoir 104 via the lumen 218 of the needle 210 and the first channel 212 of the instrument 200 using, for example, vacuum suction generated by the first pump 220. A wash solution in the second channel 214, handled by the associated second pump 222, is then drawn to the drug reservoir 104 via the lumen 218 of the needle 210 to wash away and rinse the drug reservoir 104; the waste from the wash-removal process is collected using the first waste channel 212 and its connected pump 220, as described above. The wash-removal process may be repeated as many times as necessary for effectiveness. After the final waste-removal step is complete, the drug refilling solution in the third channel 216 may be injected into the drug reservoir 104 using the associated third pump 224. As described above, during the filling/refilling process, only a single needle insertion in the fluid access refill port is required; this thus reduces the needle insertion frequency into the drug reservoir 104 of the implanted pump 100. Additionally, if a refill procedure involves directing multiple fluids to the implanted pump 100, a single needle insertion using the instrument 200 may suffice. In one embodiment, a drug container 226 (e.g., a vial) directly connects to the third channel 216 such that the drug flows out of the container 226 into the drug reservoir 104 via the third channel 216, without the risks of contamination or other human error introduced when performing the intermediate step of delivering drug from a vial to the drug reservoir 104 using a needle or other delivery means.

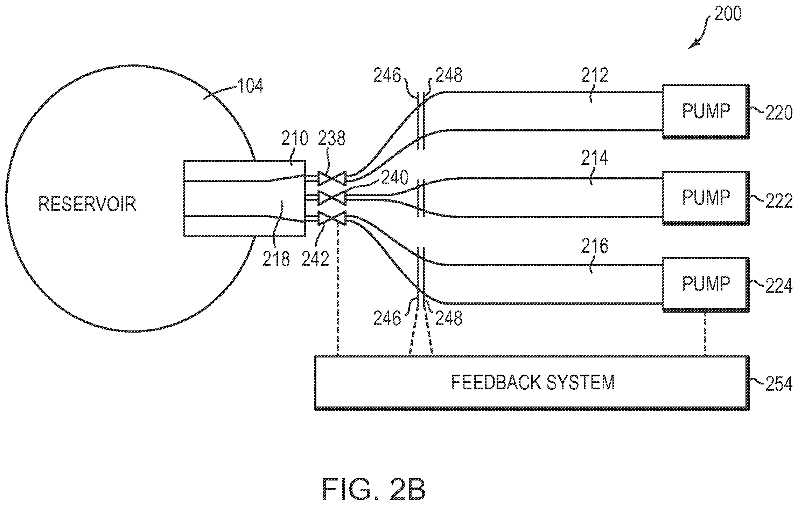

In some embodiments, as illustrated in FIG. 2B, the fluid channels 212, 214, 216 connect to the needle 210 via valves 238, 240, 242. Alternatively, or additionally, the valves 238, 240, 242 may be integral with the fluid channels 212, 214, 216, and may be located anywhere along the channels. Prior to a filling/refilling process, all three valves 238, 240, 242 are initially closed when the needle 210 is inserted into the drug reservoir 104 through the refill port so that the outlet of the needle 210 is in fluid communication with the drug chamber 104 but is isolated from the rest of the system by the valves. In a first step, valve 238 connected to the first waste channel 212 is opened and any fluid that is left in the reservoir 104 is removed using, for example, suction by activating the associated pump 220. In a second step, valve 238 is then closed and valve 240 is opened to pump a wash solution into the drug chamber 104 via the solution channel 214; the waste from the wash step is collected using the method described in step one. In one embodiment, the suction and wash steps are alternately and repeatedly performed (by alternately closing and opening valves 238 and 240). Alternatively, valve 238 may be left open during step two such that the suction is left on to perform a continuous rinse of the drug reservoir 104. In either case, after the final waste-removal step is complete, valves 238 and 240 are closed and valve 242 is opened to fill the reservoir 104 with the drug solution via the solution channel 216 (step three).

In various embodiments, the refill instrument 200 includes flow sensors 246 and/or pressure sensors 248 to monitor and control the flow rate and/or pressure, respectively, of the fluid injection and suction in each channel 212, 214, 216. For example, flow sensors 246, based upon thermal effects, time-of-flight, and/or pressure, as explained further below, may be employed within the channels to sense the fluidic flow. In one embodiment, flow sensors 246 based on thermal effects use a resistive heater to locally heat the fluid flowing in proximity to the sensors 246. The temperature of the flowing fluid in the channel then provides an indication of the flow rate. For example, time-of-flight flow sensors 246 generate a tracer pulse in the fluid flowing within the channel, and then measure the time that it takes for this pulse to traverse a certain distance. This measured time is defined as the "time of flight" and corresponds to the linear fluid velocity, which may be translated into a volumetric flow rate. In another embodiment, flow sensors 246 utilize pressure sensing and are employed within the fluid channel to measure the pressure therein and, based thereon, to increase or reduce the fluid flow rate through the channels when necessary. The pressure-based flow sensors 246 may function in any of a variety of ways; for example, capacitive, piezoresistive, and piezoelectric implementations, among others known to those of ordinary skill in the art, may all be employed advantageously. In various embodiments, if one or more pressure sensors 248 are placed inside the channels 212, 214, 216, operations of the channels associated pumps 220, 222, 224 may be adjusted to maintain an optimal pressure or pressure range during the filling/refilling process and thus avoid excess pressure, prevent damage to the pumps, and unwanted ejection of drug into the patient.

A critical set of values that define the upper and lower bounds of the safe range of pressure and/or flow rate may be determined before the filling/refilling process. If the pressure and/or flow rates exceed or fall below the set critical values during the filling/refilling procedure, an alarm system may be turned on and/or a feedback system may be initiated to control the pressure and flow rate such that the pressure and flow rates inside the fluid channels 212, 214, 216 and/or the drug reservoir 104 remain within safe operational values; this prevents drug expulsion or damage to the instrument 200 and/or the drug chamber 104.

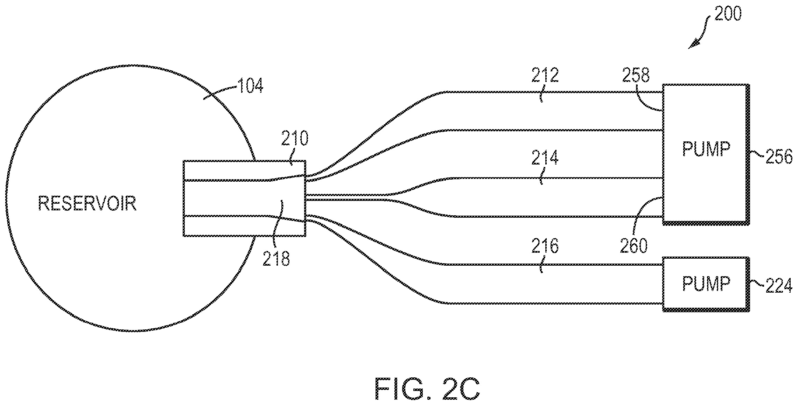

The pumps 220, 222, 224 that are in fluid communication with the fluid channels 212, 214, 216 and handle the waste solution, wash solution and filling/refilling solutions may be standard mechanical pumps (e.g., gear, diaphragm, peristaltic, syringe, etc.) or pneumatic systems that create vacuum or adjust pressure in the individual channels. Pneumatic systems may include, but are not limited to, vacuum generators, air compressors, pneumatic motors, and pneumatic actuators, etc. The pumps 220, 222, 224 work cooperatively with the flow sensors 246, pressure sensors 248 and/or valves 238, 240, 242 to control the flow rate and/or pressure in the fluid channel 212, 214, 216 during the refilling process. In addition, the volume of fluid may be metered to prevent overfilling. If the drug reservoir 104 reaches full capacity such that the internal pressure begins to rise, pumps 220, 222, 224 may adjust the pressure such that fluid is injected less into the reservoir 104 and/or aspirated more from the reservoir 104. In one embodiment, the fluid injection pressure is monitored and maintained below a critical value when a liquid is infused into the drug reservoir 104 pneumatically. If the pressure exceeds the critical value, a pressure-release valve (not shown) may be used to reduce the pressure inside the channel. In another embodiment, if the liquid is infused using a mechanical pump, the pressure may be monitored and controlled by a pressure sensor 248 disposed at the point of highest hydraulic pressure; a feedback system 254 (e.g., control circuitry) may then be used to prevent the pressure at this point from exceeding the critical value. In general, the feedback system 254 is typically implemented on a printed circuit board ("PCB") and may interface with the pumps 220, 222, 224 associated with the fluid channel 212, 214, 216, the flow sensors 246, the pressure sensors 248 and/or the valves 238, 240, 242. In response to the measured flow rates and/or pressures in the fluid channel, the feedback system 254 takes corrective action in order to ensure that the flow rate and/or pressure of the drug delivered through the channels remains within the critical range. For example, when receiving pressure data indicating that the pressure inside the fluid channel is too high, the feedback system 254 can automatically adjust operation of the pumps 220, 222, 224 and/or valves 238, 240, 242 to avoid excess pressure and/or maintain an optimal pressure or pressure range, thus preventing harm to the patient. The number of pumps used to drive the fluids in the channels may be different from (e.g., less than) the number of channels. For example, as depicted in FIG. 2C, one pump 256 may be used to connect to the first waste and second wash channels 212, 214: while one outlet 258 of the pump 256 generates suction such that the fluid in the drug reservoir 104 is removed, another outlet 260 of the pump 256 exerts a pressure to drive the fluid flow into the drug reservoir 104.

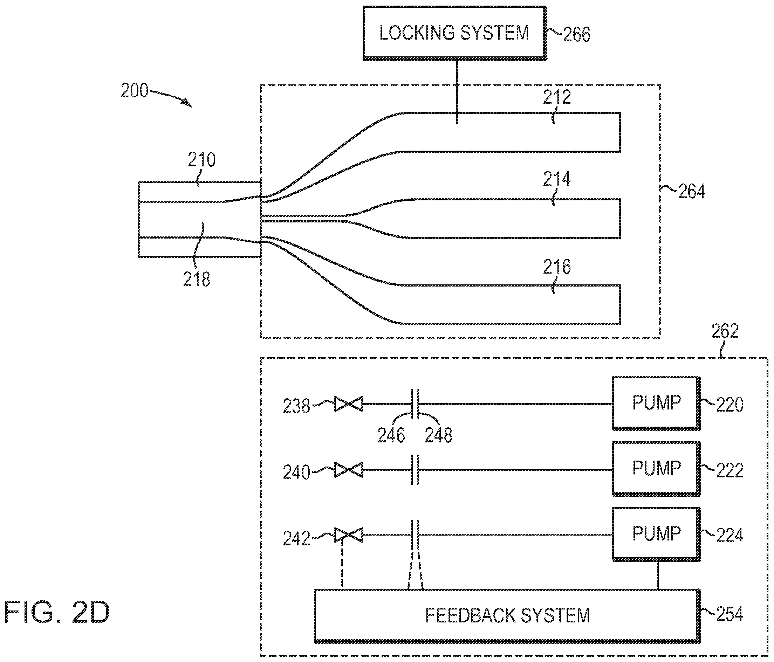

The feedback system 254, pumps 220, 222, 224 that are associated with the fluid channel, the flow sensors 246 and/or the pressure sensors 248, the valves 238, 240, 242, and/or the channels 212, 214, 216 may be implemented as a single unit or as multiple components. Referring to FIG. 2D, in one embodiment, the pumps 220, 222, 224, sensors 246, 248 and valves 238, 240, 242 are integrated with the feedback system 254 to form a base unit 262 while the fluid channels 212, 214, 216 are combined (e.g., into a single cartridge structure) to form a filling/refilling kit 264. The filling/refilling kit 264 may be mated with the base unit 262 and the needle 210 at the time of use. In such implementations, the filling/refilling kit 264 may be a single-use disposable component that is replaced each time a new reservoir is filled. The filling/refilling kit 264 may be provided to the end-user as a pre-filled kit or empty. In the case of an empty filling kit, fluid may be manually transferred to the kit 264 or the procedure may be performed automatically by the base unit 262.

In some embodiments, an electronic or mechanical locking system 266 is employed to prevent a user from injecting an unapproved fluid into the reservoir. The locking system may be based on, for example, electronic tags (e.g., RFID, barcodes, etc.) that are associated with the filling/refilling kit 264 described above. If improper tags are sensed, the instrument 200 may be programmed to prevent filling.

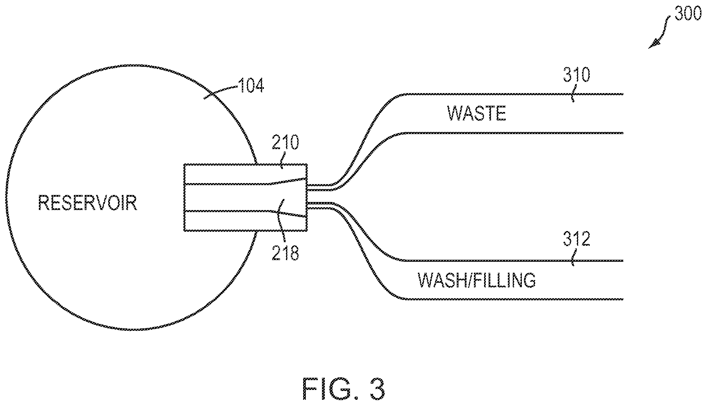

Although described above is a three-channel system, one of ordinary skill in the art will understand that systems may have different numbers of channels that ultimately terminate in the needle 210 and are within the scope of the current invention. For example, fewer independently controlled fluid channels may be utilized in the current invention. Referring to FIG. 3, an exemplary system 300 uses two independently controlled fluid channels 310, 312, where the wash channel and filling channel are merged in a single channel 312. Therefore, instead of using a dedicated wash solution to rinse the drug reservoir 104, the drug solution itself can be used. As a result, two independent fluid channels 310, 312 - channel 312 for infusing the drug and channel 310 for aspirating liquid out of the reservoir 104 - may suffice.

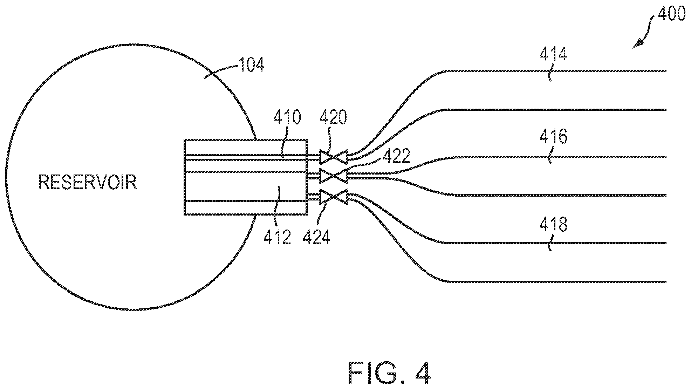

As described above, the fluid channels may be interfaced to a flow control system ultimately terminating in a needle 210, which is used to pierce the access port and access the fluid reservoir 104. In one embodiment, the needle includes one lumen and all fluids from the channels travel in and out of the single lumen, as depicted in FIG. 2A-2D and 3. In another embodiment, with reference to FIG. 4, the needle includes two lumens 410, 412, which provide two parallel, isolated paths for fluid to flow between the channels 414, 416, 418 and the drug reservoir 104. One of these lumens, i.e., lumen 410 may be dedicated for aspiration and the other lumen, i.e., lumen 412, may be used to infuse liquid (e.g., wash and drug solutions). During the filling/refilling procedure, all valves except valve 420 may be closed and the fluid in the reservoir 104 is removed via flowing through lumen 410. The reservoir 104 is then washed by opening valve 422 and pumping the wash solution through lumen 412. The waste from the washing step may then be removed through lumen 410. Finally, after the reservoir 104 is completely washed, valves 420 and 422 may be closed and valve 424 is opened to fill/refill the drug reservoir via lumen 412. Again, channels 416 and 418 may be merged to a single channel and the drug may serve as a rinse solution; this merged channel thus delivers the same drug solution during the filling/refilling procedure.

The terms and expressions employed herein are used as terms and expressions of description and not of limitation, and there is no intention, in the use of such terms and expressions, of excluding any equivalents of the features shown and described or portions thereof. Accordingly, the described embodiments are to be considered in all respects as only illustrative and not restrictive.

## Claims

1. A tool (200) for refilling an implantable pump (100) having a reservoir (104), the tool (200) comprising:
a disposable refilling kit comprising first and second independent fluid channels (212, 214) connectable at first ends thereof, via a needle (210), to the reservoir (104);
a base unit comprising a single pump (256), a sensor (246) for monitoring at least one parameter relating to flow through the fluid channels, and feedback circuitry for controlling flow through the fluid channels based on the at least one monitored parameter, wherein the base unit (262) is removably connectable to second ends of the fluid channels;
**characterised in that**,
it further comprises
a locking system (266) associated with the refilling kit for preventing injection of an unapproved fluid into the reservoir (104).

2. The tool of claim 1, wherein there are at least two independent fluid channels (212, 214, 216), the pump being configured to generate suction through a first one of the channels and to drive a fluid through a second one of the channels.

3. The tool of claim 2, where there is a third independent fluid channel connected to the pump, wherein the pump is configured to drive a second fluid through the third fluid channel.

4. The tool of claim 1, further comprising a sensor (246, 248) associated with each of the channels (212, 214, 216) for monitoring at least one parameter relating to liquid flowing therethrough.

5. The tool of claim 4, wherein the sensor (246) is a flow sensor and the parameter is a flow rate of the liquid.

6. The tool of claim 4, wherein the sensor (248) is a pressure sensor and the parameter is pressure in the channels.

7. The tool of claim 4, further comprising feedback circuitry for controlling flow through the fluid channels based on the monitored parameter.

8. The tool of claim 7, further comprising first and second valves (238, 240, 242), responsive to the governing circuitry, for controlling fluid flow through the first and second fluid channels, respectively.

9. The tool of claim 1, wherein the needle (210) has separate lumens each fluidly coupled to one of the fluid channels.

## Patentansprüche

1. Werkzeug (200) zum Nachfüllen einer implantierbaren Pumpe (100), die ein Reservoir (104) aufweist, wobei das Werkzeug (200) Folgendes umfasst:
einen Einweg-Nachfüllsatz, der einen ersten und einen zweiten unabhängigen Fluidkanal (212, 214) umfasst, die an ersten Enden derselben, über eine Nadel (210), mit dem Reservoir (104) verbunden werden können;
eine Basiseinheit, die eine einzelne Pumpe (256), einen Sensor (246) zum Überwachen mindestens eines Parameters in Bezug auf einen Strom durch die Fluidkanäle, und Rückkopplungsschaltungen zum Regeln des Stroms durch die Fluidkanäle auf Grundlage des mindestens einen überwachten Parameters umfasst, wobei die Basiseinheit (262) abnehmbar mit zweiten Enden der Fluidkanäle verbunden werden kann,
**dadurch gekennzeichnet, dass** es ferner Folgendes umfasst:
ein Verriegelungssystem (266), das mit dem Nachfüllsatz verknüpft ist, zum Verhindern einer Injektion eines nicht zugelassenen Fluids in das Reservoir (104).

2. Werkzeug nach Anspruch 1, wobei es mindestens zwei unabhängige Fluidkanäle (212, 214, 216) gibt, wobei die Pumpe dafür konfiguriert ist, einen Sog durch einen ersten der Kanäle zu erzeugen und ein Fluid durch einen zweiten der Kanäle zu treiben.

3. Werkzeug nach Anspruch 2, wobei es einen dritten unabhängigen Fluidkanal gibt, der mit der Pumpe verbunden ist, wobei die Pumpe dafür konfiguriert ist, ein zweites Fluid durch den dritten Fluidkanal zu treiben.

4. Werkzeug nach Anspruch 1, das ferner einen Sensor (246, 248) umfasst, der mit jedem der Kanäle (212, 214, 216) verknüpft ist, zum Überwachen mindestens eines Parameters bezüglich einer Flüssigkeit, die durch dieselben strömt.

5. Werkzeug nach Anspruch 4, wobei der Sensor (246) ein Durchflusssensor ist und der Parameter eine Durchflussmenge der Flüssigkeit ist.

6. Werkzeug nach Anspruch 4, wobei der Sensor (248) ein Drucksensor ist und der Parameter ein Druck in den Kanälen ist.

7. Werkzeug nach Anspruch 4, das ferner Rückkopplungsschaltungen zum Regeln eines Stroms durch die Fluidkanäle auf Grundlage des überwachten Parameters umfasst.

8. Werkzeug nach Anspruch 7, das ferner ein erstes und ein zweites Ventil (238, 240, 242) umfasst, die auf die Regelungsschaltungen ansprechen, zum Regeln eines Fluidstroms durch den ersten beziehungsweise den zweiten Fluidkanal.

9. Werkzeug nach Anspruch 1, wobei die Nadel (210) gesonderte Lumina aufweist, die jeweils mit einem der Fluidkanäle fluidisch verbunden sind.

## Revendications

1. Outil (200) pour recharger une pompe implantable (100) ayant un réservoir (104), l'outil (200) comprenant :
un kit de remplissage jetable comprenant des premier et deuxième canaux de fluide (212, 214) indépendants pouvant être connectés à leurs premières extrémités, *via* une aiguille (210), au réservoir (104) ;
une unité de base comprenant une pompe unique (256), un capteur (246) pour surveiller au moins un paramètre relatif à l'écoulement à travers les canaux de fluide, et des circuits de rétroaction pour contrôler l'écoulement à travers les canaux de fluide sur la base du au moins un paramètre surveillé, dans lequel l'unité de base (262) peut être connectée de manière amovible à des deuxièmes extrémités des canaux de fluide ;
**caractérisé en ce qu'**il comprend en outre
un système de verrouillage (266) associé au kit de remplissage pour empêcher l'injection d'un fluide non approuvé dans le réservoir (104).

2. Outil selon la revendication 1, dans lequel il y a au moins deux canaux de fluide (212, 214, 216) indépendants, la pompe étant configurée pour générer une aspiration à travers un premier des canaux et pour entraîner un fluide à travers un deuxième des canaux.

3. Outil selon la revendication 2, dans lequel il y a un troisième canal de fluide indépendant relié à la pompe, dans lequel la pompe est configurée pour entraîner un deuxième fluide à travers le troisième canal de fluide.

4. Outil selon la revendication 1, comprenant en outre un capteur (246, 248) associé à chacun des canaux (212, 214, 216) pour surveiller au moins un paramètre relatif à un liquide s'écoulant à travers celui-ci.

5. Outil selon la revendication 4, dans lequel le capteur (246) est un capteur de débit et le paramètre est un débit du liquide.

6. Outil selon la revendication 4, dans lequel le capteur (248) est un capteur de pression et le paramètre est la pression dans les canaux.

7. Outil selon la revendication 4, comprenant en outre des circuits de rétroaction pour contrôler l'écoulement à travers les canaux de fluide sur la base du paramètre surveillé.

8. Outil selon la revendication 7, comprenant en outre des première et deuxième vannes (238, 240, 242), sensibles aux circuits de régulation, pour contrôler l'écoulement de fluide à travers le premier et le deuxième canal de fluide, respectivement.

9. Outil selon la revendication 1, dans lequel l'aiguille (210) a des lumières séparées, chacune étant couplée de manière fluidique à l'un des canaux de fluide.
